# FASCICULE DE BREVET EUROPEEN

(11) **EP 2 408 809 B1**
(45) Date de publication et mention de la délivrance du brevet: **30.10.2013**
(21) Numéro de dépôt: 10716555.7
(22) Date de dépôt: 18.03.2010
(51) Int. Cl.: C07K 14/65, C07K 14/655, A61K 38/16

(54) **PEPTIDES MODULANT L'ACTIVITE DE L'IGF-1 ET LEURS APPLICATIONS**
PEPTIDE, DIE DIE AKTIVITÄT VON IGF-1 MODULIEREN; UND DIE ANWENDUNGEN DER PEPTIDE
PEPTIDES MODULATING THE ACTIVITY OF IGF-1 AND THE APPLICATIONS OF SAID PEPTIDES

(30) Priorité: 19.03.2009 FR 0951754
(43) Date de publication de la demande: 25.01.2012
(73) Titulaire: Université Pierre et Marie Curie, 75005 Paris (FR)
(72) Inventeur: CARELLI, Claude, F-92150 Suresnes (FR); LARON, Zvi, 52960 Ramat Efal (IL); MAOR, Gila, 26360 Kiriat-motzkin (IL)
(74) Mandataire: Chajmowicz, Marion
(86) Numéro de dépôt international: PCT/FR2010/050490
(87) Numéro de publication internationale: WO 2010/106294

(56) Documents cités:
- C R R GRACE ET AL.: "Novel sst2-selective somatostatin agonists. Three-dimensional consensus structure by NMR" JOURNAL OF MEDICINAL CHEMISTRY., vol. 49, no. 15, 2006, pages 4487-4496, XP002556039 AMERICAN CHEMICAL SOCIETY. cité dans la demande
- A PANDIT ET AL.: "Self-assembly of the octapeptide lanreotide and lanreotide--based derivatives; the role of the aromatic residues" JOURNAL OF PEPTIDE SCIENCE., vol. 14, no. 1, 2008, pages 66-75, XP002523807 JOHN WILEY AND SONS LTD. cité dans la demande

### SEQUENCE LISTING

<110> Université Pierre et Marie Curie
<120> PEPTIDES MODULANT L'ACTIVITE DE L'IGF-1 ET LEURS APPLICATIONS
<130> B855
<160> 8
<170> PatentIn version 3.3
<210> 1
   <211> 7
   <212> PRT
   <213> artificial séquence
<220>
   <223> SEQ ID NO: 1_ formule (I)
<220>
   <221> MISC_FEATURE
   <222> (1) .. (1)
   <223> Xaa est une séquence comprenant 10 à 12 acides aminés choisie parmi: Phe-Gly-Ser-Arg-Lys-Phe-Ser-Tyr-Lys-Ala (a), Asn+(a), Ser-Asn+(a), ou une séquence substantiellement homologue
<220>
   <221> MISC_FEATURE
   <222> (2)..(2)
   <223> Xaa est un résidu lysine, arginine ou histidine
<220>
   <221> MISC_FEATURE
   <222> (5) .. (5)
   <223> Xaa est un résidu thréonine ou lysine
<220>
   <221> MISC_FEATURE
   <222> (6).. (6)
   <223> Xaa est un résidu aspartate ou glutamate
<220>
   <221> MISC_FEATURE
   <222> (7) .. (7)
   <223> Xaa est une séquence comprenant 5 à 7 acides aminés choisie parmi: Val-Thr-Thr-Ser-Glu, Val-Thr-Thr-Ser-Glu-Leu, Val-Thr-Thr-Ser-Glu-Leu-Gly, ou une séquence substantiellement homologue
<400> 1
   Xaa Xaa Phe Trp Xaa Xaa Xaa
   1               5
<210> 2
   <211> 24
   <212> PRT
<220>
   <223> SEQ ID NO: 2_ formule (II) _ P70
<400> 2
   Ser Asn Phe Gly Ser Arg Lys Phe Ser Tyr Lys Ala Lys Phe Trp Thr
   1               5                   10                  15
   Asp Val Thr Thr Ser Glu Leu Gly
               20
<210> 3
   <211> 10
   <212> PRT
   <213> artificial sequence
<220>
   <223> SEQ ID NO: 3
<400> 3
   Phe Gly Ser Arg Lys Phe Ser Tyr Lys Ala
   1               5                   10
<210> 4
   <211> 11
   <212> PRT
   <213> artificial sequence
<220>
   <223> SEQ ID NO: 4
<400> 4
   Asn Phe Gly Ser Arg Lys Phe Ser Tyr Lys Ala
   1               5                   10
<210> 5
   <211> 12
   <212> PRT
   <213> artificial sequence
<220>
   <223> SEQ ID NO: 5
<400> 5
   Ser Asn Phe Gly Ser Arg Lys Phe Ser Tyr Lys Ala
   1               5                   10
<210> 6
   <211> 5
   212> PRT
<220>
   <223> SEQ ID NO: 6
<400> 6
   Val Thr Thr Ser Glu
   1               5
<210> 7
   <211> 6
   <212> PRT
   <213> artificial sequence
<220>
   <223> SEQ ID NO: 7
<400> 7
   Val Thr Thr Ser Glu Leu
   1               5
<210> 8
   <211> 7
   <212> PRT
   <213> artificial sequence
<220>
   <223> SEQ ID NO: 8
<400> 8
   Val Thr Thr Ser Glu Leu Gly
   1               5

## Description

L'invention concerne des peptides modulateurs de l'IGF-1.

### Arrière-plan technique :

Le processus cancéreux, qui se traduit par une prolifération cellulaire non contrôlée, est souvent l'expression d'une surexpression de facteurs de croissance et/ou de leurs récepteurs dans une même cellule (Moyse *et al.* 1985, Cassoni *et al.* 2006, Hanahan *et al.* 2000).

De nombreux facteurs, agissant en réseau, sont responsables de la progression du cycle cellulaire et de la prolifération cellulaire. En particulier, un groupe de ces facteurs, dits de « croissance », sont représentés par des peptides capables de stimuler l'entrée en phase S et, in fine, la division cellulaire. C'est le cas des IGF (Insulin-like Growth Factors) (Pardee 1989, Cross *et al.* 1991).

L'IGF-1, en particulier, synthétisée après stimulation par l'hormone de croissance (Growth Hormone, GH), agit comme une hormone endocrine, et peut même être vue comme la véritable hormone de croissance (Laron, 2001). Agissant également selon un mécanisme paracrine/autocrine, cette hormone a un rôle important chez l'enfant dont elle stimule la croissance, tandis que chez l'adulte elle augmente l'anabolisme.

Le récepteur spécifique de cette hormone (IGF-1R) est exprimé sur de nombreux types cellulaires différents et donc plusieurs tissus de l'organisme dépendent de l'action de L'IGF-1 : on peut citer le foie, les reins, le poumon, les muscles, le tissu osseux et le cartilage, mais aussi le tissu nerveux. Par ailleurs l'IGF-1 a des effets proches de l'insuline puisque il peut se lier - avec une faible activité - au récepteur de l'insuline, il régule le développement des cellules nerveuses, et régule la synthèse de l'ADN.

La liaison de l'IGF-1 au récepteur induit un signal intracellulaire qui stimule la croissance et la prolifération cellulaire (Perona, 2006). Cette activité fait de l'IGF-1 à la fois un puissant inhibiteur de l'apoptose (mort cellulaire programmée) et un activateur de la synthèse protéique (Yanochko *et al* 2006, Colòn *et al* 2007, Inoue *et al* 2005). Enfin, dans certaines conditions, l'IGF-1 peut avoir le double rôle d'inducteur de l'apoptose et de stimulateur de la prolifération cellulaire (Fu *et al* 2007, Rabinovsky 2004 a).

Le réseau de signalisation de l'IGF a un rôle crucial dans la progression tumorale, y compris dans le phénomène métastatique (Hofmann *et al* 2005), et de nombreux travaux expérimentaux mais aussi épidémiologiques tendent à établir une corrélation significative entre un taux important de IGF-1 et/ou de son récepteur et le risque accru, voire l'établissement d'une prolifération cancéreuse (Vella *et al* 2001, Talapatra *et al* 2001, Kucab *et al* 2003, Kambhampati *et al* 2005, Bjorndahl *et al* 2005, Gennigens *et al* 2006, Velcheti *et al* 2006, Yanochko et al 2006, Sisci *et al* 2007, Samani *et al* 2007). D'une façon générale, le rôle de l'axe GH/IGF-1 dans l'oncogenèse et surtout dans la stimulation de la progression tumorale est donc bien établi, comme le montrent de nombreux travaux épidémiologiques et une étude très récente montrant qu'une déficience congénitale de l'IGF-1 semble protéger du développement d'un cancer (Sheva *et al* 2007).

Des études cliniques sur des molécules inhibitrices de l'activité IGF montrent le grand intérêt que peut avoir leur utilisation dans différents types de cancer, leur effet thérapeutique agissant en synergie avec la chimiothérapie et l'irradiation (Min *et al* 2005, Carmiraud *et al* 2005, Chinnavian *et al* 2006, Wu *et al* 2006, Deutsh *et al* 2005, Warshamana-Greene *et al* 2005). L'IGF-1 étant un médiateur de l'hormone de croissance, sa production peut être retardée ou inhibée en amont de la production de l'hormone de croissance (GH), par la somatostatine.

La Somatostatine *(Somatotrope Release Inhibiting Factor,* ou SRIF), sécrétée dans l'hypothalamus, est un antagoniste naturel de la GHRH (neuropeptide stimulant la synthèse de GH). En particulier, en inhibant la GH, le SRIF a un effet d'inhibition sur la synthèse de l'IGF-1. Mais le SRIF a également une action à la périphérie, notamment en agissant comme une hormone digestive en inhibant, par exemple, la sécrétion des hormones gastro-intestinales et pancréatiques.

La capacité du SRIF et de ses analogues synthétiques à inhiber la prolifération cellulaire et à déclencher la mort cellulaire par des mécanismes indirects ou directs est mise à profit dans plusieurs protocoles thérapeutiques visant différents types de cancers en clinique humaine. De plus, le SRIF est capable d'inhiber l'angiogénèse, ce qui représente un intérêt potentiel en clinique pour le contrôle indirect de la croissance des tumeurs (Ferjoux *et al* 2000). En effet, les tumeurs s'alimentent par la néovascularisation (angiogénèse), or les analogues de SRIF, qui inhibent la prolifération cellulaire, ont également un effet indirect d'inhibition sur la sécrétion de facteurs de croissance comme le Vascular Endothelial Growth Factor (VEGF), tout en réduisant la chimiotaxie monocytaire (Dasgupta 2004, Garcia de la Torre *et al.* 2002).

Cependant, l'utilisation d'analogues du SRIF n'est possible que dans les cancers exprimant les récepteurs du SRIF. Ainsi l'efficacité des analogues du SRIF utilisés en clinique a été explicitement démontrée seulement dans le traitement des tumeurs neuroendocrines, gastroentéropancréatiques, du cerveau, du sein, de la prostate et du poumon (Ferjoux *et al* 2000). Par ailleurs, il ne faut pas oublier que le taux et le profil d'expression de récepteurs du SRIF varient énormément dans les différents carcinomes.

Des molécules analogues de la somatostatine, inhibitrices du «système IGF» (Pawlikowski M. *et al.,* 2004), ont été proposées. Il s'agit notamment du BIM 23A387, de l'Octreotide et du Lanreotide (deux peptides 8-mères). Ces molécules modulent l'expression du récepteur ou de son ligand, et sont des candidats intéressants dans la thérapie de différents types de cancer et de l'acromégalie. Autre exemple, le ligand de synthèse bispécifique, BIM-23244 (Rani C., 2004, Rani C., 2006, Pandit A., 2008), est capable d'inhiber la sécrétion de l'hormone de croissance dans les adénomes somatotropes.

### Résumé de l'invention :

Les inventeurs proposent maintenant de nouveaux peptides capables de moduler les effets de l'IGF-1. Ces peptides comprennent la séquence de 20 à 24 acides aminés (I) ci-après :

**Pₐ-X₁-Phe-Trp-X₂-X₃-P_{b}** (SEQ ID NO: 1) (I)

dans laquelle :
- Pₐ représente une séquence comprenant 10 à 12 acides aminés choisie parmi les séquences :
   Phe-Gly-Ser-Arg-Lys-Phe-Ser-Tyr-Lys-Ala (SEQ ID NO: 3)
   Asn-Phe-Gly-Ser-Arg-Lys-Phe-Ser-Tyr-Lys-Ala (SEQ ID NO: 4)
   Ser-Asn-Phe-Gly-Ser-Arg-Lys-Phe-Ser-Tyr-Lys-Ala (SEQ ID NO: 5)
- X₁ est un résidu lysine, arginine ou histidine ;
- X₂ est un résidu thréonine ou lysine ;
- X₃ est un résidu aspartate ou glutamate ; et
- P_{b} représente une séquence comprenant 5 à 7 acides aminés choisie parmi les séquences :
   Val-Thr-Thr-Ser-Glu (SEQ ID NO: 6)
   Val-Thr-Thr-Ser-Glu-Leu (SEQ ID NO: 7)
   Val-Thr-Thr-Ser-Glu-Leu-Gly (SEQ ID NO: 8)

Il est également compris dans l'invention un peptide résistant à la protéolyse dérivé de la séquence (i) par modifications de l'extrémité N-terminale sous forme d'un acétyle, et/ou de l'extrémité C-terminale sous forme d'un groupe amide et/ou d'au moins une liaison peptidique modifiée et remplacée par une liaison (CH2-NH), une liaison (NH-CO), une liaison (CH2-O), une liaison (CH2-S), une liaison (CH2-CH2), une liaison (CO-CH2), une liaison (CHOH-CH2), une liaison (N-N), une liaison alcéne-E ou encore une liaison CH=CH), ou un peptide homologue défini comme étant dérivé de la séquence (I) par une ou plusieurs substitution(s) conservative(s) et présentant plus de 80% des acides aminés identiques à la séquence (I).

Ces peptides sont utiles pour moduler, de préférence stimuler, une prolifération cellulaire *in vitro.*

L'invention a également pour objet une composition pharmaceutique comprenant au moins un tel peptide en tant que principe actif, en association avec un ou plusieurs excipients physiologiquement acceptables.

La présente invention a aussi pour objet un peptide, tel que défini ici, pour une utilisation à titre de médicament.

### Description détaillée de l'invention :

### Définitions :

Le terme "modulation" se réfère à une activité inhibitrice et/ou activatrice des peptides de l'invention sur les fonctions de l'IGF-1.

Par "inhibition" on entend tout effet inhibiteur sur la synthèse de IGF-1 et/ou l'expression de IGF-1R incluant une inhibition et/ou une régulation négative réduisant l'activité, l'activation, les fonctions et/ou l'expression de IGF-1. Par "activation" on entend tout effet potentialisant, stimulant ou activant la synthèse de IGF-1 et/ou l'expression de IGF-1R incluant une activation et/ou une régulation positive de l'activité, des fonctions et/ou de l'expression de IGF-1. Un effet inhibiteur et/ou activateur d'un peptide est étudié par des mesures de l'expression de IGF-1 et/ou de IGF-1R. Il est également possible d'étudier l'inhibition et/ou l'activation induite par un peptide de l'invention au moyen de culture cellulaire exprimant IGF-1 et/ou IGF-1R.

Le terme "patient" se réfère à tout animal humain ou non humain, de préférence un mammifère, incluant male, femelle, adulte et/ou enfant nécessitant un traitement modulant la synthèse de IGF-1 et/ou de l'expression du récepteur de IGF-1.

Le terme "traitement" ou "thérapie" comprend aussi bien un traitement curatif qu'un traitement prophylactique d'une maladie. Un traitement curatif est défini par un traitement aboutissant à une guérison ou un traitement allégeant, améliorant et/ou éliminant, réduisant et/ou stabilisant les symptômes d'une maladie ou la souffrance qu'elle provoque. Un traitement prophylactique comprend aussi bien un traitement aboutissant à la prévention d'une maladie qu'un traitement réduisant et/ou retardant l'incidence d'une maladie ou le risque qu'elle survienne.

L'expression "substitution conservative" exprime tout remplacement d'un résidu acide aminé par un autre, sans altération de la conformation générale ni de la fonction du peptide. La substitution conservative inclut, sans s'y limiter, le remplacement par un acide aminé ayant des propriétés similaires (comme par exemple la forme, la polarité, le potentiel de liaison hydrogène, l'acidité, la basicité, l'hydrophobicité et autres). Les acides aminés ayant des propriétés similaires sont bien connus dans l'art. Par exemple, l'arginine, l'histidine et la lysine sont des acides aminés hydrophiles basiques et peuvent être interchangeables. De la même façon, l'isoleucine, un acide amine hydrophobe, peut être remplacé par une leucine, une méthionine ou une valine. Les acides aminés hydrophiles neutres pouvant se substituer les uns les autres incluent l'asparagine, la glutamine, la sérine et la thréonine. Par "substitué" et "modifié " l'invention comprend les acides aminés ayant été altéré ou modifié par rapport à un acide aminé trouvé dans la nature. L'expression "séquence substantiellement homologue" comprend toute séquence soumise à une ou plusieurs substitution(s) conservative(s).

Ainsi, dans le contexte de la présente invention, une substitution conservative est connue dans l'art comme une substitution d'un acide aminé par un autre ayant des propriétés similaires. Des exemples de substitutions conservatives sont donnés dans le tableau 1 ci-dessous :

**Tableau 1. Substitutions conservatives I**

| **Caractéristique de la chaîne latérale** | **Acide aminé** |
|---|---|
| Non polaire | G A P I L V |
| Polaire non chargée | C S T M N Q |
| Polaire chargée | D E K R |
| Aromatique | H F W Y |
| autre | N Q D E |

Selon Lehninger, 1975, les acides aminés conservatifs peuvent également être groupés comme illustré dans le tableau 2 ci-dessous :

**Table 2. Substitutions conservatives II**

| **Caractéristique de la chaîne latérale** | | **Acide aminé** |
|---|---|---|
| Non polaire (hydrophobe) | | |
| | A. Aliphatique : | A L I V P |
| | B. Aromatique : | F W |
| | C. Contenant un sulfuryle : | M |
| | D. Autre | G |
| | | |
| Polaire non chargée | | |
| | A. Hydroxyle : | S T Y |
| | B. Amides : | N Q |
| | C. Sulfhydryle : | C |
| | D. Autre | G |
| Chargée positivement (basique) | | K R H |
| Chargée négativement (acide) | | D E |

Encore une autre alternative de substitution conservative est présentée dans le tableau 3 ci-dessous:

**Table 3. Substitutions Conservatives III**

| **Résidu d'origine** | **Exemple de substitution** |
|---|---|
| Ala (A) | Val (V), Leu (L), Ile(I) |
| Arg (R) | Lys (K), Gln (Q), Asn (N) |
| Asn (N) | Gln (Q), His (H), Lys (K), Arg (R) |
| Asp (D) | Glu (E) |
| Cys(C) | Ser (S) |
| Gln (Q) | Asn (N) |
| Glu (E) | Asp (D) |
| His (H) | Asn (N), Gln (Q), Lys (K), Arg (R) |
| Ile(I) | Leu (L), Val (V), Met (M), Ala (A), Phe (F) |
| Leu (L) | Ile(I), Val (V), Met (M), Ala (A), Phe (F) |
| Lys (K) | Arg (R), Gln (Q), Asn (N) |
| Met (M) | Leu (L), Phe (F), Ile(I) |
| Phe (F) | Leu (L), Val (V), Ile(I), Ala (A) |
| Pro (P) | Gly (G) |
| Ser (S) | Thr (T) |
| Thr (T) | Ser (S) |
| Trp (W) | Tyr (Y) |
| Tyr (Y) | Trp (W), Phe (F), Thr (T), Ser (S) |
| Val (V) | Ile(I), Leu (L), Met (M), Phe (F), Ala (A) |

### Préparation des peptides :

Les peptides conformes à l'invention peuvent être préparés par toutes les techniques classiques de synthèse peptidique, à savoir notamment par synthèse chimique ou recombinaison génétique. Dans un mode de réalisation préféré, les peptides sont obtenus par synthèse chimique. De préférence encore, les peptides sont obtenus soit par condensation successive des résidus d'acides aminés dans l'ordre requis, soit par condensation des résidus sur un fragment préalablement formé et contenant déjà plusieurs acides aminés dans l'ordre approprié, ou encore par condensation de plusieurs fragments préalablement préparés, en prenant soin de protéger, au préalable, toutes les fonctions réactives portées par les résidus d'acides aminés, excepté les fonctions amine et carboxyle engagées dans la liaison peptidique lors de la condensation, et notamment par la technique de synthèse en phase solide de Merrifield.

### Propriétés des peptides :

Les peptides de l'invention sont capables de moduler les effets de l'IGF-1. Ces peptides comprennent, ou sont constitués, de la séquence de 20 à 24 acides aminés (I) ci-après :

**Pₐ-X₁-Phe-Trp-X₂-X₃-P_{b}** (SEQ ID NO :1) (I)

dans laquelle :
- Pₐ représente une séquence comprenant 10 à 12 acides aminés choisie parmi les séquences :
   Phe-Gly-Ser-Arg-Lys-Phe-Ser-Tyr-Lys-Ala (SEQ ID NO: 3)
   Asn-Phe-Gly-Ser-Arg-Lys-Phe-Ser-Tyr-Lys-Ala (SEQ ID NO: 4)
   Ser-Asn-Phe-Gly-Ser-Arg-Lys-Phe-Ser-Tyr-Lys-Ala (SEQ ID NO: 5)
- X₁ est un résidu lysine, arginine ou histidine ;
- X₂ est un résidu thréonine ou lysine ;
- X₃ est un résidu aspartate ou glutamate ; et
- P_{b} représente une séquence comprenant 5 à 7 acides aminés choisie parmi les séquences :
   Val-Thr-Thr-Ser-Glu (SEQ ID NO: 6)
   Val-Thr-Thr-Ser-Glu-Leu (SEQ ID NO: 7)
   Val-Thr-Thr-Ser-Glu-Leu-Gly (SEQ ID NO: 8)
Il est également compris dans l'invention, un peptide résistant à la protéolyse dérivé de la séquence (I) par une ou plusieurs modifications chimiques ou un peptide substantiellement homologue dérivé de la séquence (I) par une ou plusieurs substitution(s) conservative(s).

De préférence, X₁ est un résidu lysine, X₂ est un résidu thréonine, et X₃ est un résidu aspartate.

Dans un mode de réalisation préféré, le peptide comprend la séquence d'acides aminés suivante :

### SNFGSRKFSYKAKFWTDVTTSELG (SEQ ID NO : 2)

De préférence, les extrémités N- et/ou C-terminales sont protégées de la protéolyse. Par exemple, l'extrémité N-terminale peut être sous la forme d'un acétyle, et/ou l'extrémité C-terminale peut être sous la forme d'un groupe amide. Toute modification visant à rendre résistants les peptides de l'invention est envisagée, par exemple des peptides dans lesquels au moins une liaison peptidique est modifiée et remplacée par une liaison (CH2NH), une liaison (NHCO), une liaison (CH2-O), une liaison (CH2-S), une liaison (CH2CH2), une liaison (CO-CH2), une liaison (CHOH-CH2), une liaison (N-N), une liaison alcène-E ou encore une liaison -CH=CH.

Tous les peptides modifiés chimiquement pour résister à la protéolyse sont compris dans la présente invention.

Par exemple le peptide ci-dessus désigné P70 peut être sous la forme
Acétyl-SNFGSRKFSYKAKFWTDVTTSELG-Amide

L'invention prévoit également des peptides substantiellement homologues dérivés de la séquence (I) par une ou plusieurs substitution(s) conservative(s). Deux séquences d'acides aminés sont dites « substantiellement homologues »ou « substantiellement similaires » quant un ou plusieurs résidus acides aminés sont remplacés par un ou plusieurs résidus similaires d'un point de vue biologique, ou lorsque plus de 80% des acides aminés sont identiques, ou de manière préférée lorsque plus de 90% des résidus sont identiques (fonctionnellement identique). Les séquences similaires ou homologues sont de préférence identifiées par alignement en utilisant, par exemple, le programme « Pileup » GCG (Genetics Computer Group, manuel de programme pour le Package GCG, *Version* 7, Madison, Wisconsin), ou tout autre programme connu par l'homme du métier (BLAST, FASTA, etc.)

Conformément à l'Invention, tous les résidus acides aminés des peptides peuvent appartenir à la série L (lévogyre). Toutefois, il est également possible, quelque soit l'acide aminé de la séquence (I), de substituer la forme D (dextrogyre) à la forme L. En particulier il est envisagé que certains acides aminés - lesquels sont situés en positions 11 à 18 dans la séquence (I) et notamment le tryptophane en position 15- appartiennent à la série D, de manière à conférer aux peptides une meilleure résistance à l'action des peptidases.

Avantageusement, les peptides conformes à l'invention sont liés de façon covalente, au niveau de leur extrémité C-terminale ou d'un résidu lysine, à une ou plusieurs molécule(s) de polyéthylène glycol (PEG), et notamment d'un PEG de PM 1500 ou 4000, propre à augmenter la demi-vie plasmatique et à diminuer la dose thérapeutique à administrer. Cette liaison peut être réalisée comme décrit par Abuchowski et al. (J. Biol. Chenu., 1977,252 : 3582-3586). Selon un autre mode de réalisation, les peptides de l'invention sont introduits dans des polymères ou des co-polymères biodégradables formant des microsphères, par exemple le poly(D,L-lactide-co-glycolide (US2007/0184015, SoonKap Hahn et al).

Les peptides de l'invention, très différents de la somatostatine endogène (native) ou des peptides précédemment décrits, présentent une conformation moléculaire en « épingle à cheveux » (ou ß-hairpin) très stable en milieu physiologique comme en milieu hydrophobe. Avantageusement, aucune condition du milieu ne peut induire une transition de la conformation peptidique en hélice α.

En outre, cette conformation en « épingle à cheveux » permet de bien exposer, juste au centre du coude formé par le repliement de la séquence peptidique, deux acides aminés (Phe-Trp) sur les quatre acides aminés contigus (Phe-Trp-Lys-Thr) considérés dans SRIF comme majeurs pour la liaison aux récepteurs. Sans vouloir être liés par une hypothèse, les inventeurs pensent que dans la séquence des peptides de l'invention, une attraction de type électrostatique entre l'acide aminé en position 13 et l'acide aminé en position 17 permet de reproduire une sorte de « coude », en faisant assumer au peptide entier une conformation de type ß (conformation ß de type VIII).

Les peptides de l'invention présentent un effet bi-phasique. Ils sont ainsi capables, selon la concentration utilisée, de stimuler ou d'inhiber la production de IGF-1 ainsi que l'expression de son récepteur (IGF-1 R). *In vitro,* ces effets ont pour conséquence soit de stimuler soit d'inhiber la prolifération cellulaire.

Cet effet bi-phasique, ou « effet en cloche » d'une molécule biologique, qui en fonction de la dose utilisée se traduit en un effet agoniste ou antagoniste, est bien connu pour d'autres molécules et dans différents systèmes biologiques (enzymes [Gamage et al. 2003, He et al. 2003], canaux ioniques et co-transporteurs [Arias et al. 1996, Lombardi et al. 2001, Borst et al. 2002, Incerpi et al. 2003], transporteurs [Henry et al. 2002], récepteurs de tyrosine kinases [Leiser et al. 1986, Schlessinger 1988], récepteurs couplés aux protéines G (GPCRs), neurotransmetteurs, hormones et chemokines [Winding et al. 1993, Chidiac et al. 1996, Bronnikov et al. 1999, Cuthbert 2003, Hornigold et al. 2003, Griffin et al. 2003, Fuh et al. 1992, Talmadge 1998] et en particulier concernant la GnRH et ses analogues [Browning J.Y et al.1983, Ho M.N. et al.1997, Barbarino A et al.1982, Imai, A et al.1993, Kang, Sung K et al.2000 et 2001, Gründker C et al. 2003, Leung P.C.K et al. 2003, Bhasin S. et al. 2008].

Les peptides de l'invention sont ainsi utiles pour stimuler *in vitro* une prolifération cellulaire, par exemple dans le cadre de cultures de chondrocytes, de kératocytes ou d'hépatocytes ou pour la préparation de tissus d'intérêt médical.

Compte tenu de leurs propriétés remarquables, les peptides conformes à l'invention trouvent de nombreuses applications, *in vitro* et *in vivo,* notamment en médecine humaine ou en médecine vétérinaire.

Les peptides de l'invention peuvent être utilisés à une dose adaptée
- soit pour stimuler la synthèse de IGF-1 et/ou l'expression du récepteur de l'IGF-1 ;
- soit pour inhiber la synthèse de IGF-1 et/ou l'expression du récepteur de l'IGF-1.

La stimulation de la synthèse de l'IGF-1 et/ou de l'expression de son récepteur est recherchée notamment dans les cas suivants :
- lorsque les peptides sont utilisés en tant que substitut de l'hormone de croissance. Les peptides sont en effet particulièrement utiles dans toutes les indications thérapeutiques liées à un déficit complet ou partiel, congénital ou acquis, de l'hormone de croissance endogène ou de l'IGF-1, et notamment dans le traitement des retards structuraux chez l'enfant ou chez l'adulte. D'autres applications thérapeutiques comprennent par exemple le syndrome de Turner, le syndrome de Prader-Willi, l'insuffisance rénale chronique. Les peptides de l'invention sont en particulier utiles dans les affections dans lesquelles une augmentation de la masse musculaire peut être recherchée comme les états cachectiques liés au syndrome d'immuno-déficience aquise (SIDA) ou au cancer. Les peptides sont également utiles dans le domaine vétérinaire, notamment dans l'élevage bovin, porcin, de moutons, de chevaux, etc.
- dans le traitement thérapeutique du syndrome métabolique, tel que l'obésité ou le diabète de type II (notamment en abaissant le taux des lipides sanguins et en améliorant le métabolisme du glucose), ainsi que pour stimuler ou restaurer les défenses immunitaires, en particulier chez les sujets âgés ou immunodéprimés, ou pour le traitement de maladies autoimmunes (telles que diabète de type I) et/ou neuro-dégénératives (telles que la sclérose en plaque).
pour stimuler la survie des cellules neuronales (effet anti-apoptotique), la différenciation ou le développement neuronal, la régénération des nerfs moteurs ou sensitifs, la synaptogénèse et la remyélinisation. En stimulant la production d'IGF-1, les peptides de l'invention sont donc utilises en tant qu'agents neurotrophiques pour stimuler la neurogénèse et/ou comme agents neuroprotecteurs (en particulier en cas d'épisode ischémique).

L'inhibition de la synthèse de l'IGF-1 et/ou de l'expression de son récepteur est recherchée notamment dans les cas suivants :
- lorsque les peptides sont utilisés en tant que substitut de la somatostatine, comme par exemple dans des pathologies pour lesquelles l'octréotide et le lantréotide sont prescrits. L'effet biologique alors recherché peut, entre autre, être : une inhibition de la sécrétion hypophysaire de GH, une inhibition de la sécrétion biliaire, des sécrétions endocrines et exocrines pancréatiques et gastro-intestinales. Les peptides peuvent par exemple être utilisés en tant que substitut de la somatostatine pour le traitement de la maladie d'Alzheimer.
- dans le traitement de l'acromégalie, en particulier chez des patients pour lesquels la chirurgie et/ou le traitement par radiation n'est pas adapté. Le traitement par les peptides de l'invention a alors pour but de normaliser les taux sériques d'IGF-1.
- dans le traitement d'un cancer, en particulier mais sans s'y limiter, pour le traitement du cancer du sein, du pancréas ou de la prostate ou pour le traitement d'un adénome hypophysaire, ou d'une tumeur endocrine digestive. Les peptides de l'invention peuvent également traiter les métastases (en particulier les métastases gastro-intestinales). Il est également envisagé d'utiliser les peptides de l'invention à une dose inhibitrice pour traiter des symptomes (diarrhée, flushs) associés à des tumeurs endocrines digestives.

### Compositions pharmaceutiques :

Les peptides de l'invention peuvent être administrés par toute voie d'administration connue, incluant les voies : systémique (parentérale, intraveineuse...), orale, rectale, topique, sous-cutané, intrapulmonaire (voir Agu et al, 2001) et intranasale. Selon un mode de réalisation préféré, les peptides de l'invention sont administrés par voie intranasale.

*In vitro,* un exemple de dose adaptée pour stimuler la synthèse de IGF-1 et/ou l'expression du récepteur de l'IGF-1 est une dose à une concentration de l'ordre de 10⁻⁷M, et un exemple de dose adaptée pour inhiber la synthèse de IGF-1 et/ou l'expression du récepteur de l'IGF-1 est une dose à une concentration d'au moins 10⁻⁶M.

Les doses correspondantes *in vivo* sont ajustées de manière classique en fonction de l'effet souhaité, et de la pathologie visée :
Une dose efficace pour stimuler *in vivo* la synthèse de l'IGF-1 et/ou l'expression de son récepteur inclut une concentration de moins de 100 µg par jour, de préférence de 10 µg à 100 µg par jour. De manière préférée, la dose journalière efficace est comprise entre 10 µg et 50 µg. Une dose efficace pour inhiber *in vivo* la synthèse de l'IGF-1 et/ou l'expression de son récepteur inclut une concentration d'au moins 300 µg par jour, de préférence de 300 µg à 3 mg par jour. De manière préférée, la dose journalière efficace est comprise entre 1 mg et 3 mg, de manière encore plus préférée la dose journalière efficace est comprise entre 2 mg et 3 mg. Pour une administration intranasale ou intrapulmonaire, ou encore pour des formulations à libération prolongée, les doses stimulatrices et/ou inhibitrices à prévoir peuvent être de 10 à 100 fois inférieures. Ces administrations peuvent être répétées sur de longues périodes, de préférence sur une période allant de 3 à 6 mois.

La présente Invention sera mieux comprise à l'aide du complément de description qui suit et qui se réfère à des exemples de mise en évidence de l'activité biologique d'un peptide conforme à l'Invention.

La séquence du peptide de l'invention utilisé dans les exemples et les figures de la présente demande - nommé P70 - consiste en la séquence en acides aminés suivante :
Acetyl-SNFGSRKFSYKAKFWTDVTTSELG-amide (SEQ ID NO : 2)

Il doit être bien entendu, toutefois, que ces exemples sont donnés uniquement à titre d'illustrations de l'objet de l'Invention et n'en constituent en aucune manière une limitation.

### LEGENDE DES FIGURES

La FIGURE 1 est un graphe montrant l'effet de P70 sur l'incorporation de [H³] Thymidine dans l'ADN.

La FIGURE 2 est un graphe montrant l'effet de P70 sur l'expression de l'IGF-1R évalué par mesure de la densité optique.

La FIGURE 3 montre l'effet de P70 sur l'expression de l'IGF-1R sur une culture primaire de chondrocytes de mandibule de souris nouveau-né, par immunohistochimie.

La FIGURE 4 montre l'effet de P70 sur la synthèse de l'IGF-1 sur une culture primaire de chondrocytes de mandibule de souris nouveau-né, par immunohistochimie La FIGURE 5 montre l'effet de P70 sur la production de Collagène de type II sur une culture primaire de chondrocytes de mandibule de souris nouveau-né, par immunohistochimie.

La FIGURE 6 montre l'effet de P70 sur le processus de chondrogénèse sur une culture primaire de chondrocytes de mandibule de souris nouveau-né. Les flèches montrent les nodules de formation de cartilage.

### EXEMPLES :

### EXEMPLE 1 : Effet d'un peptide de l'invention sur la prolifération cellulaire

Les effets biologiques des peptides de l'invention sont démontrés dans un modèle de culture primaire de chondrocytes de mandibule de souris nouveau-né.

Afin d'évaluer la capacité du peptide à faire proliférer les cellules, les inventeurs ont mis en oeuvre un test capable de montrer l'incorporation de [H³] Thymidine au cours de la duplication de l'ADN selon la méthode de Kurz *et al.,* 1997. Ce test a été adapté au modèle utilisé par les inventeurs, à savoir une culture primaire de chondrocytes embryonnaires de souris.

Les chondrocytes, obtenus par dissociation de condyles de mandibule de souris (MCDC) ont été incubés dans un milieu de culture aptes à permettre la chondrogénèse, en particulier, 5 x 10⁵ cellules/ml ont été incubés avec 1µCi [³H]-thymidine/ml de milieu (Amersham, code TRA120, stock 1mCi/ml, activité spécifique 5 Ci/mmol) pendant 3 heures à 37°C dans un milieu DMEM (Dulbecco's modified Eagle medium) ne contenant pas de sérum de voeu foetal (FCS) mais supplémenté avec 100 µg/ml d'acide ascorbic,1 mmol/L de chlorure de calcium, 10 mmol/L de ß-glyceroposphate, et antibiotiques. Le même milieu, dans lequel ont été ajoutés pendant 2 minutes 5µl de [³H]-thymidine, est utilisé comme contrôle négatif. Après avoir jeté le surnageant et lavé 2 fois les cellules avec une solution physiologique tamponnée à pH 7.0 (PBS), les cellules sont lavées deux fois (pendant 5 minutes) avec du méthanol. Ensuite les cellules sont lavées trois fois avec une solution froide de 10% d'acide tri chlore acétique (TCA). Après avoir lavé encore deux fois les cellules avec du DDW, les cellules sont mises dans 200 µl de NaOH 0.3M pendant 15 minutes. Les cellules ont été ensuite transférées dans 3 ml d'un liquide à scintillation neutralisé avec 200 µl de HCl 0.3M. Enfin, après avoir été fortement vortexées, les échantillons contenant les cellules ont été placées dans un compteur bêta pendant 1 minute.

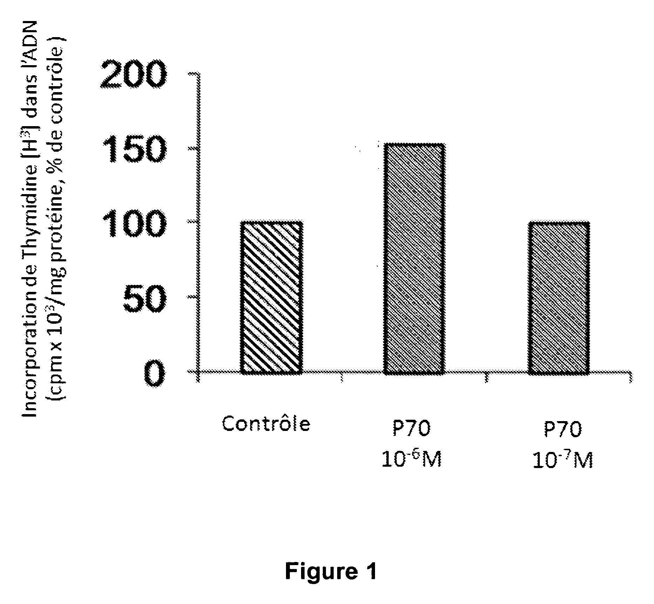

Les résultats obtenus montrent (Figure 1) que le peptide P70 a un effet de stimulation de la prolifération des chondrocytes de 53% supérieure au contrôle non traité (chondrocytes n'ayant été incubés avec aucun peptide ni autre facteur de croissance), à la concentration la plus forte (10⁻⁶M), alors que cet effet de stimulation n'est pas observé à une concentration plus faible (10⁻⁷M).

### EXEMPLE 2: Effet d'un peptide de l'invention sur la synthèse de l'IGF1 et sur l'expression du récepteur de l'IGF1

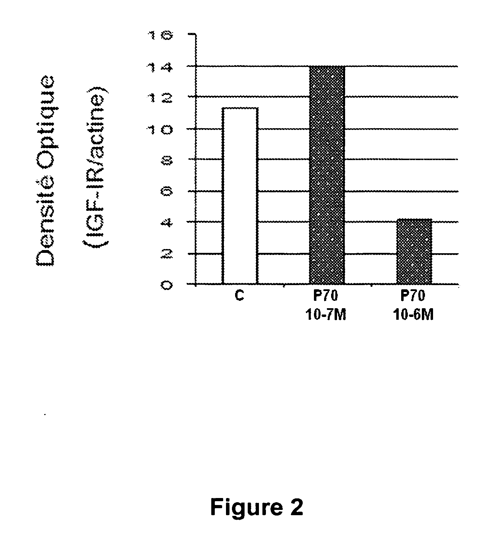

Les inventeurs ont étudié l'effet de P70 sur la synthèse de l'IGF-1 et sur l'expression de son récepteur (IGF-1R) au moyen de chondrocytes en culture. Les inventeurs ont pu montrer que le peptide a un effet important de stimulation de la production de l'IGF-1 et de l'expression de son récepteur (IGF-1R) quand les chondrocytes sont incubés avec une faible concentration de peptide (10⁻⁷ M) **(****Figure 2****).**

En revanche, à une concentration plus élevée (10⁻⁶M), le peptide inhibe sensiblement la production de l'IGF-1 et de son récepteur.

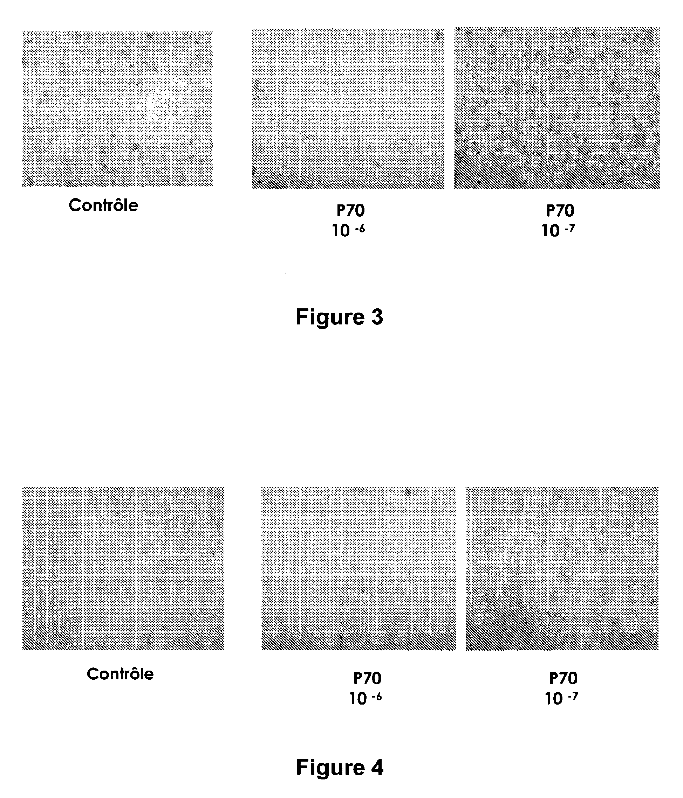

Par immunohistochimie (IHC) sur une culture primaire de chondrocytes de 5 jours **(****Figure** 3) les inventeurs montrent également que le peptide P70 inhibe l'expression du récepteur d'IGF-1 à 10⁻⁶ M (inhibition de 36,8% à une forte concentration de peptide) tandis qu'il stimule l'expression de ce même récepteur à 10⁻⁷ M (augmentation de 23,8 % à une faible concentration de peptide). De même **(****Figure 4****),** P70 inhibe la synthèse de IGF-1 à 10⁻⁶M (inhibition de 30% à une haute concentration de peptide) et il la stimule à 10⁻⁷M (augmentation de 90 % à une faible concentration de peptide).

### EXEMPLE 3 : Effet d'un peptide de l'invention sur la différenciation cellulaire

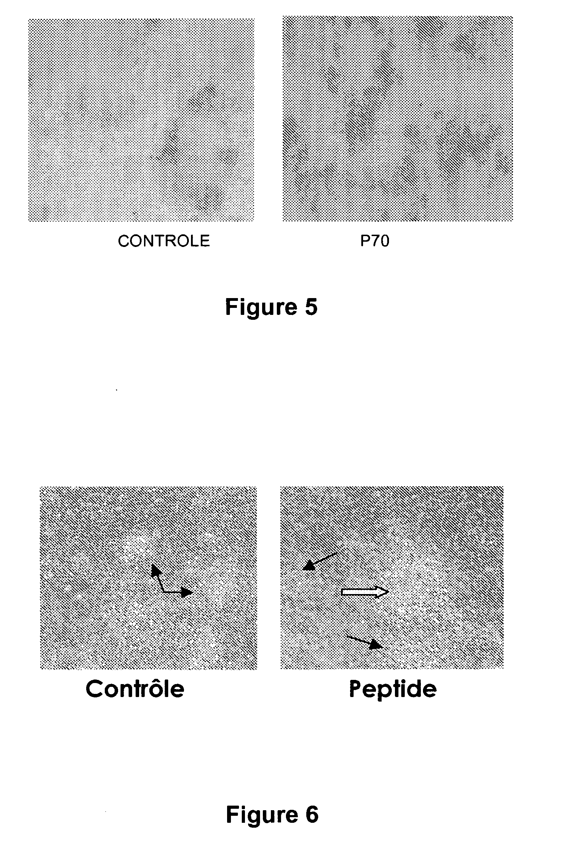

Les chondrocytes d'embryons de souris obtenus selon le même protocole que les exemples précédents, ont été mis en culture et incubés à une concentration de 10⁻⁷ M du peptide P70. Au 5^{ème} jour de culture, les inventeurs montrent par immunochimie (marquage par anticorps spécifiques anti-collagène de type II) que le peptide stimule significativement une production de collagène de type II, composant majeur de la matrice extracellulaire servant à la production du cartilage articulaire **(****Figure 5****).** En effet, une augmentation de 150% est observée par rapport au témoin (control) non traité.

Dans la **Figure 6** l'effet du peptide, P70 à la concentration de 10⁻⁷M, a été comparé à des chondrocytes non traités (contrôle). Il apparaît clairement qu'au 7ème jour d'incubation, en présence du peptide, on assiste à une accélération de la formation de nodules de cartilage. Le développement des nodules cartilagineux (volume/culture) est accéléré de 85%.

### CONCLUSION

Le peptide P70 présente un profil bi-phasique :
- à faible concentration (10⁻⁷M) :
   - il stimule la différentiation cellulaire, notamment en accélérant les premières phases de la chondrogenèse (voir Fig. 5 - expression du Collagène de type II- et Fig. 6 -chondrogenèse-)
   - il stimule la production de l'IGF-1 et l'expression du récepteur de l'IGF-1 (voir Fig. 2 -Densité optique- et Fig. 3 -immunohistochimie-) et la synthèse de IGF-1 (voir Fig. 4 - immunohistochimie).
- à haute concentration (10⁻⁶M) :
   - il stimule la prolifération cellulaire (voir Fig. 1 -incorporation de thymidine tritiée-)
   - il inhibe la production de IGF-1 (voir Fig. 4 -immunohistochimie-) et l'expression du récepteur de l'IGF-1 (voir Fig. 2 -Densité optique- et Fig. 3 -immunohistochimie-)

### Références bibliographiques

- Arias HR: (1996) Agonist self-inhibitory binding site of the nicotinic acetylcholine receptor. J Neurosci Res, 44:97-105.
- Agu RU, Ugwoke MI, Armand M, Kinget R, Verbeke N. The lung as a route for systemic delivery of therapeutic proteins and peptides. Respir Res. 2001;2(4):198-209. Epub 2001 Apr 12
- Barbarino A, De Marinis L., Mancini A., Giustacchini M.,Alcini A.E. (1982) Biphasic effect of estradiol on luteinizing hormone response to gonadotropin-releasing hormone in castrated men, Metabolism 31(8):755-8
- Bhasin S, Heber D, Steiner B, Peterson M, Blaisch B, Campfield L A, Swerdloff R.S. (2008) Hormonal effects of GnRH agonist in the human male : II. Testosterone enhances gonadotrophin suppression induced by GnRH agonist. Clinical Endocrinologyy 20: 119-128
- Bjorndahl M. et al. (2005). "Insulin-like growth factor 1 and 2 induce lymphangiogenesis in vivo. PNAS USA 102, 15593-15598.
- Borst P, Elferink RO: Mammalian ABC transporters in health and disease. Annu Rev Biochem 2002, 71:537-592.
- Bronnikov GE, Zhang SJ, Cannon B, Nedergaard J (1999) A dual component analysis explains the distinctive kinetics of cAMP accumulation in brown adipocytes. J Biol Chem, 274:37770-37780.
- Browning J.Y., R. D'Agata, A. Steinberger, H.E. Grotjan Jr and E. Steinberger (1983) Biphasic effect of gonadotropin-releasing hormone and its agonist analog (HOE766) on in vitro testosterone production by purified rat Leydig cells, Endocrinology, Vol 113, 985-991
- Camiraud A. et al. (2005). "Inhibition of insulin-like growth factor-1 receptor signalling enhances growth-inhibitory and proapoptotic effects of gefitinib (Iressa) in human breast cancer cells". Breast Cancer Res. 7, R570-579.
- Cassoni P. et al. (2006). "Grelin and cortistatin in lung cancer:expression of peptides and related eceptors in human primary tymors in vitro effect on the H345 small cell carcinoma cell line". J. Endocrinol. Invest. 29, 781-790.
- Chidiac P, Nouet S, Bouvier M (1996) Agonist-induced modulation of inverse agonist efficacy at the beta 2-adrenergic receptor. Mol Pharmacol, 50:662-669.
- Chinnavian P. et al. (2006). "Radiation and new molecular agents, pert II: targeting HDAC, HSPçà, IGF-1R, PI3K, and Ras". Semin. Radiat. Oncol. 16, 59-64.
- Colòn E. et al. (2007).Insulin-like growth factor-1 is an important antiapoptotic factor for rat leydig cells during postnatal development. Endocrinology 148, 128-139.
- Cross M., T.M. Dexter (1991). "Growth factors in development, transformation, and tumorigenesis". Cell 64, 271-280
- Cuthbert AW: (2003) Benzoquinolines and chloride secretion in murine colonic epithelium. Br J Pharmacol, 138:1528-1534.
- Dasgupta 2004, (2004) « Somatostatin analogues : multiple roles in cellular proliferation, neoplasia, and angiogenesis » Pharmacol Ther 102 (1): 61-85
- Deutsch E. et al. (2005). "New strategies to interfere with radiation response : "biomodulation" of radiation therapy". Cancer Radiother. 9, 69-76.
- Ferjoux G., C. Bousquet, P. Cordelier et al. (2000). "Signal transduction of somatostatin receptors negatively controling cell proliferation". J. Physiol. (Paris) 94, 205-210.
- Fu P. et al. (2007). "Insulin-like growth factor induces apoptosis as well as proliferation in LIM1215 colon cancer cells". J. Cell. Biochem. 100, 56-68.
- Fuh G, Cunningham BC, Fukunaga R, Nagata S, Goeddel DV, Wells JA (1992) Rational design of potent antagonists to the human growth hormone receptor. Science, 256:1677-80.
- Gamage NU, Duggleby RG, Barnett AC, Tresillian M, Latham CF, Liyou NE, McManus ME, Martin JL (2003) Structure of a human carcinogen-converting enzyme, SULT1A1. Structural and kinetic implications of substrate inhibition. J Biol Chem, 278:7655-7662
- Garcia de la Torre N, Wass JA, Turner HE, (2002) « Antiangiogenic effects of somatostatin analogues" Clin Endocrinol 2002 57(4): 425-441 Review
- Gennigens C., C. Menetrier-Caux, J.P. Droz (2006). "Insulin-like growth factor (IGF) family and prostate cancer". Crit. Rev. Oncol. Hematol. 58,124-145
- Griffin MT, Hsu JC, Shehnaz D, Ehlert FJ: Comparison of the pharmacological antagonism of M2 and M3 muscarinic receptors expressed in isolation and in combination. Biochem Pharmacol 2003, 65:1227-1241.
- Gründker C. and G. Emons (2003) Role of gonadotropin-releasing hormone (GnRH) in ovarian cancer. Reproductive Biology and Endocrinology 1:65
- Hanahan D. , R.A. Weinberg (2000). "The hallmarks of cancer". Cell 100, 57-70.
- He YA, Roussel F, Halpert JR (2003) Analysis of homotropic and heterotropic cooperativity of diazepam oxidation by CYP3A4 using site-directed mutagenesis and kinetic modeling. Arch Biochem Biophys, 409:92-101.
- Henry ER, Bettati S, Hofrichter J, Eaton WA (2002) A tertiary two-state allosteric model for hemoglobin. Biophys Chem, 98:149-164.
- Ho M.N., Delgado C.H., Owens G.A., Steller M.A. (1997) Insulin-like growth factor-II participates in the biphasic effect of a gonadotropin-releasing hormone agonist on ovarian cancer cell growth, Fertility and Sterility Volume 67, Number 5, May 1997 , pp. 870-876(7)
- Hofmann F., C. Garcia-Echeverria (2005). "Blocking insulin-like growth factor-1 receptor as a strategy for targeting cancer". DDT 10, 1041-1047.
- Hornigold DC, Mistry R, Raymond PD, Blank JL, Challiss RA (2003) Evidence for cross-talk between M2 and M3 muscarinic acetylcholine receptors in the regulation of second messenger and extracellular signal-regulated kinase signalling pathways in Chinese hamster ovary cells. Br J Pharmacol, 138:1340-1350.
- Imai, A, lida, K, Tamaya, T (1993) Gonadotropin-releasing hormone has a biphasic action on aromatase activity through protein kinase C in granulosa cells. Int-J-Fertil-Menopausal-Stud. 38(1): 50-56
- Incerpi S, D'Arezzo S, Marino M, Musanti R, Pallottini V, Pascolini A, Trentalance A (2003) Short-term activation by low 17beta-estradiol concentrations of the Na+/H+ exchanger in rat aortic smooth muscle cells: physiopathological implications. Endocrinology, 144:4315-4324.
- Inoue A. et al. (2005). "Insulin-growth factor-1 stimulated DNA replication in mouse endometrial stromal cells". J. Reprod. Dev. 51, 305-313.
- Kambhampati S. et al. (2005). "Growth factors involved in prostate carcinogenesis". Front. Biosci. 10, 1355-1367.
- Kang, Sung K.; Cheng, Kwai W.; Nathwani, Parimal S.; Choi, Kyung-Chul; Leung, Peter C. K.(2000) Autocrine Role of Gonadotropin-Releasing Hormone and Its Receptor in Ovarian Cancer Cell Growth. Endocrine. 13(3):297-304
- Kang S.K., C-J Tai, P S. Nathwani and P.C. K. Leung (2001) Differential regulation of two forms of Gonadotrophin-related hormone messenger ribonucleic acid in human granulosa-luteal cells. Endocrinology 142: 1182-1192
- Kucab J.E., S.E. Dunn (2003). "Role of IGF-1R in mediating breast cancer invasion and metastasis". Breast Dis. 17, 41-47
- Kurz B, Schünke M, (1997) « Articular chondrocytes and synoviocytes in culture : influence of antioxidants on lipid peroxidation and proliferation." Ann. Anat. 1997, 179: 439-446
- Laron Z. (2001). "Insulin-like growth factor 1 (IGF-1) : a growth hormone". J. Clin. Pathol. Mol. Pathol. 54, 311-316.
- Lehninger, (1975) Biochemistry, Second Edition, Worth Publishers, Inc. New-York: NY., pp. 71-77.
- Leiser J, Conn PM, Blum JJ: (1986) Interpretation of dose-response curves for luteinizing hormone release by gonadotropinreleasing hormone, related peptides, and leukotriene C4 according to a hormone/receptor/effector model. Proc Natl Acad Sci U S A, 83:5963-7.
- Leung P.C.K., C.K.Cheng and Z. Xiao-Ming (2003) Multi-factorial role of GnRH-I and GnRH-II in the human ovary. Molecular and Cellular Endocrinology 202: 145-153
- Lombardi G, Dianzani C, Miglio G, Canonico PL, Fantozzi R: Characterization of ionotropic glutamate receptors in human lymphocytes. Br J Pharmacol 2001, 133:936-944.
- Min Y. et al. (2005). "Insulin_like growth factor I receptor blockade enhances chemotherapy and radiation responses and inhibits tumor growth in human gastric cancer xenografts". Gut 54, 591-60.
- Moyse E. et al. (1985). "Somatostatin receptors in human growth hormone and prolactin-secreting pituitary adenomas". J. Clin. Endocrinol. Metab. 61, 98-103.
- Pandit A., fay N., Border L., Valéry C., Cherif-Cheikh R., Robert B., Artzner F., Paternoster M. J. (2008) Pept. Sci., 14, 66-75.
- Pardee A.B. (1989). "G1 events and regulation of cell proliferation". Science 246, 603-608
- Pawlikowski M. et Malen-Mucha G. Current Opinion in Pharmacology (2004), 4, 608-613.
- Perona R. (2006). "Cell signaling: growth factors and tyrosine kinase receptors". Clin. Transl. Oncol. 8, 77-82I.
- Rabinovsky E.D. (2004). "The multifunctional role of IGF-1 in peripheral nerve regeneration". Neurol. Res. 26, 204-210.
- Rani C., Durrer L., Koerber S.C., Erchegyi J., Reubi J.C., Rivier J., Riek R. J. Med. Chem. (2004), 48, 523-533
- Rani C., Durrer L., Koerber S.C., Erchegyi J., Reubi J.C., Rivier J., Riek R. J. Med. Chem. (2006), 49, 4487-4496
- Samani A.A. et al. (2007). "The Role of the IGF System in Cancer Growth and Metastasis: Overview and Recent Insights". Endocrine Reviews 28, 20-47.
- Schlessinger J (1998) Signal transduction by allosteric receptor oligomerization. Trends Biochem Sci, 13:443-447.
- Shevah O., Z. Laron (2007). "Patients with congenital deficiency of IGF-1 seem protected from the development of malignancies: A preliminary report". Growth Horm IGF Res. 17, 54-57.
- Sisci D., E. Surmacz (2007). "Crosstalk between IGF Signaling and Steroid Hormone Receptors in Breast". Cancer Curr. Pharm. Des.13, 705-717.
- Talapatra S., C. B. Thmpson (2001). "Growth factor Signaling in Cell Survival: Implications for Cancer". Treatment JPET, 298, 873-878.
- Talmadge JE (1998) Pharmacodynamic aspects of peptide administration biological response modifiers. Adv Drug Deliv Rev, 241-252
- Velcheti V, Govindan R (2006). "Insulin-like growth factor and lung cancer". Journal of thoracic oncology : official publication of the International Association for the Study of Lung Cancer 1 (7): 607-10.
- Vella V. et al. (2001). "The IGF system in thyroid cancer: new concepts". J. Clin. Pathol.: Mol. Pathol. 54, 121-125
- Warshamana-Greene G.S. et al. (2005). "The insulin-like growth factor-I receptor kinase inhibitor, NVP-ADW742, sensitized small cell lung cancer cell lines to the effects of chemotherapy". Clin. Cancer. Res. 11, 1563-1571.
- Winding B, Bindslev N (1993) Desensitization and reactivation of ACh-regulated exocrine secretion in hen tracheal epithelium. Am J Physiol, 264:C342-C351.
- Wu X. Z., D. Chen, G. R. Xie (2007). "Bone marrow-derived cells: roles in solid tumor". Neoplasma 54, 1-6.
- Yanochko G.M., W. Eckhart (2006). "Type I insulin-like growth factor receptor overexpression induces proliferation and anti-apoptotic signaling in a three-dimensional culture model of breast epithelial cells". Breast Cancer Res. 8, R18.

## Revendications

1. Peptide comprenant la séquence de 20 à 24 acides aminés (I) suivante :
**Pₐ-X₁-Phe-Trp-X₂-X₃-P_{b}** (SEQ ID NO: 1) (I)
dans laquelle :
- Pₐ représente une séquence comprenant 10 à 12 acides aminés choisie parmi les séquences :
Phe-Gly-Ser-Arg-Lys-Phe-Ser-Tyr-Lys-Ala (SEQ ID NO: 3)
Asn-Phe-Gly-Ser-Arg-Lys-Phe-Ser-Tyr-Lys-Ala (SEQ ID NO: 4)
Ser-Asn-Phe-Gly-Ser-Arg-Lys-Phe-Ser-Tyr-Lys-Ala (SEQ ID NO: 5)
- X₁ est un résidu lysine, arginine ou histidine ;
- X₂ est un résidu thréonine ou lysine ;
- X₃ est un résidu aspartate ou glutamate ; et
- P_{b} représente une séquence comprenant 5 à 7 acides aminés choisie parmi les séquences :
Val-Thr-Thr-Ser-Glu (SEQ ID NO: 6)
Val-Thr-Thr-Ser-Glu-Leu (SEQ ID NO: 7)
Val-Thr-Thr-Ser-Glu-Leu-Gly (SEQ ID NO: 8), ou
un peptide résistant à la protéolyse défini comme étant dérivé de la séquence (I) par modifications de l'extrémité N-terminale sous forme d'un acétyle, et/ou de l'extrémité C-terminale sous forme d'un groupe amide et/ou d'au moins une liaison peptidique modifiée et remplacée par une liaison (CH2NH), une liaison (NHCO), une liaison (CH2-O), une liaison (CH2-S), une liaison (CH2CH2), une liaison (CO-CH2), une liaison (CHOH-CH2), une liaison (N-N), une liaison alcène-E ou encore une liaison -CH=CH, ou un peptide homologue défini comme étant dérivé de la séquence (I) par une ou plusieurs substitution(s) conservative(s) et présentant plus de 80% des acides aminés identiques à la séquence (I).

2. Peptide selon la revendication 1, dans lequel X₁ est un résidu lysine, X₂ est un résidu thréonine et X₃ est un résidu aspartate.

3. Peptide selon la revendication 2, consistant en la séquence SNFGSRKFSYKAKFWTDVTTSELG. (SEQ ID NO: 2)

4. Composition pharmaceutique comprenant un peptide selon l'une des revendications 1 à 3 en tant que principe actif, en association avec un ou plusieurs excipients pharmaceutiquement acceptables.

5. Utilisation du peptide selon l'une des revendications 1 à 3, pour moduler une prolifération cellulaire *in vitro.*

6. Peptide selon l'une des revendications 1 à 3, à titre de médicament.

7. Peptide selon l'une des revendications 1 à 3, pour une utilisation à une dose adaptée pour stimuler la synthèse de IGF-1 et/ou l'expression du récepteur de l'IGF-1.

8. Peptide selon la revendication 7, utilisé *in vivo* à une dose journalière efficace de moins de 100 µg, de préférence de 10 µg à 100 µg, de préférence encore de 10 µg et 50 µg.

9. Peptide selon l'une des revendications 1 à 3, pour une utilisation en tant que substitut de l'hormone de croissance.

10. Peptide selon l'une des revendications 1 à 3, pour une utilisation dans le traitement de l'insuffisance rénale chronique, du syndrome de Turner ou du syndrome de Prader-Willi, ou
dans le traitement thérapeutique du syndrome métabolique, de maladies autoimmunes et/ou neurodégénératives.

11. Peptide selon l'une des revendications 1 à 3, pour une utilisation en tant qu'agent neurotrophique et/ou neuroprotecteur.

12. Peptide selon l'une des revendications 1 à 3, pour une utilisation à une dose adaptée pour inhiber la synthèse de IGF-1 et/ou l'expression du récepteur de l'IGF-1.

13. Peptide selon la revendication 12, pour une utilisation *in vivo* à une dose journalière efficace d'au moins 300 µg par jour, de préférence de 300 µg à 3 mg par jour, de préférence encore de 1 mg à 3 mg, de manière encore plus préférée de 2 mg à 3 mg.

14. Peptide selon l'une des revendications 1 à 3, pour une utilisation en tant que substitut à la somatostatine.

15. Peptide selon l'une des revendications 1 à 3, pour une utilisation dans le traitement de la maladie d'Alzheimer, ou pour une utilisation dans le traitement de l'acromégalie ou du cancer, en particulier dans le traitement de métastases.

## Patentansprüche

1. Peptid, umfassend die folgende Sequenz von 20 bis 24 Aminosäuren (I):
**Pₐ-X₁-Phe-Trp-X₂-X₃-P_{b}** (SEQ ID NR: 1) (I)
in der:
- Pₐ für eine Sequenz steht, die 10 bis 12 Aminosäuren umfasst und aus den Sequenzen ausgewählt ist:
Phe-Gly-Ser-Arg-Lys-Phe-Ser-Tyr-Lys-Ala (SEQ ID NR: 3)
Asn-Phe-Gly-Ser-Arg-Lys-Phe-Ser-Tyr-Lys-Ala (SEQ ID NR: 4)
Ser-Asn-Phe-Gly-Ser-Arg-Lys-Phe-Ser-Tyr-Lys-Ala (SEQ ID NR: 5)
- X₁ ein Lysin-, Arginin- oder Histidinrest ist;
- X₂ ein Threonin- oder Lysinrest ist;
- X₃ ein Aspartat- oder Glutamatrest ist; und
- P_{b} für eine Sequenz steht, die 5 bis 7 Aminosäuren umfasst und aus den Sequenzen ausgewählt ist:
Val-Thr-Thr-Ser-Glu (SEQ ID NR: 6)
Val-Thr-Thr-Ser-Glu-Leu (SEQ ID NR: 7)
Val-Thr-Thr-Ser-Glu-Leu-Gly (SEQ ID NR: 8), oder
ein proteolyseresistentes Peptid, das als Derivat der Sequenz (I) definiert ist mit Modifikationen des N-terminalen Endes als ein Acetyl und/oder des C-terminalen Endes als eine Amidgruppe und/oder wenigstens einer Peptidbindung, die modifiziert ist und durch eine (CH2NH)-Bindung, eine (NHCO)-Bindung, eine (CH2-O)-Bindung, eine (CH2-S)-Bindung, eine (CH2CH2)-Bindung, eine (CO-CH2)-Bindung, eine (CHOH-CH2)-Bindung, eine (N-N)-Bindung, eine E-Alken-Bindung oder auch eine -CH=CH-Bindung ersetzt ist, oder ein homologes Peptid, das als Derivat der Sequenz (I) mit einer oder mehreren konservativen Substitutionen definiert ist und mehr als 80% identische Aminosäuren mit der Sequenz (I) aufweist.

2. Peptid gemäß Anspruch 1, in dem X₁ ein Lysinrest ist, X₂ ein Threoninrest ist und X₃ ein Aspartatrest ist.

3. Peptid gemäß Anspruch 2, bestehend aus der Sequenz SNFGSRKFSYKAKFWTDVTTSELG (SEQ ID NR: 2).

4. Pharmazeutische Zusammensetzung, umfassend ein Peptid gemäß einem der Ansprüche 1 bis 3 als Wirkstoff in Kombination mit einem oder mehreren pharmazeutisch verträglichen Arzneimittelträgern.

5. Verwendung des Peptids gemäß einem der Ansprüche 1 bis 3 zur Modulation einer Zellproliferation *in vitro.*

6. Peptid gemäß einem der Ansprüche 1 bis 3 als Medikament.

7. Peptid gemäß einem der Ansprüche 1 bis 3 zur Verwendung mit einer Dosis, die zur Stimulierung der IGF-1-Synthese und/oder der Expression des IGF-1-Rezeptors geeignet ist.

8. Peptid gemäß Anspruch 7, das *in vivo* mit einer wirksamen Tagesdosis von weniger als 100 µg, bevorzugt 10 µg bis 100 µg, bevorzugter 10 bis 50 µg verwendet wird.

9. Peptid gemäß einem der Ansprüche 1 bis 3 zur Verwendung als Wachstumshormon-Substitut.

10. Peptid gemäß einem der Ansprüche 1 bis 3 zur Verwendung in der Behandlung der chronischen Niereninsuffizienz, des Turner-Syndroms oder des Prader-Willi-Syndroms, oder in der therapeutischen Behandlung des metabolischen Syndroms, von Autoimmunkrankheiten und/oder neurodegenerativen Krankheiten.

11. Peptid gemäß einem der Ansprüche 1 bis 3 zur Verwendung als neurotrophes und/oder neuroprotektives Agens.

12. Peptid gemäß einem der Ansprüche 1 bis 3 zur Verwendung mit einer Dosis, die zur Hemmung der IGF-1-Synthese und/oder der Expression des IGF-1-Rezeptors geeignet ist.

13. Peptid gemäß Anspruch 12 zur *in vivo* Verwendung mit einer wirksamen Tagesdosis von wenigstens 300 µg pro Tag, bevorzugt von 300 µg bis 3 mg pro Tag, bevorzugter von 1 mg bis 3 mg, noch bevorzugter von 2 mg bis 3 mg.

14. Peptid gemäß einem der Ansprüche 1 bis 3 zur Verwendung als Substitut für Somatostatin.

15. Peptid gemäß einem der Ansprüche 1 bis 3 zur Verwendung in der Behandlung der Alzheimer-Krankheit oder zur Verwendung in der Behandlung der Akromegalie oder von Krebs, insbesondere in der Behandlung von Metastasen.

## Claims

1. A peptide comprising following sequence (1) of 20 to 24 amino acids:
**Pₐ-X₁-Phe-Trp-X₂-X₃-P_{b}**(SEQ ID NO: 1) (I)
wherein:
- Pₐ represents a sequence comprising 10 to 12 amino acids selected from the sequences:
Phe-Gly-Ser-Arg-Lys-Phe-Ser-Tyr-Lys-Ala (SEQ ID NO: 3)
Asn-Phe-Gly-Ser-Arg-Lys-Phe-Ser-Tyr-Lys-Ala (SEQ ID NO: 4)
Ser-Asn-Phe-Gly-Ser-Arg-Lys-Phe-Ser-Tyr-Lys-Ala (SEQ ID NO: 5)
- X₁ is a lysine, arginine or histidine residue;
- X₂ is a threonine or lysine residue;
- X₃ is an aspartate or glutamate residue; and
- P_{b} represents a sequence comprising 5 to 7 amino acids selected from the sequences:
Val-Thr-Thr-Ser-Glu (SEQ ID NO: 6)
Val-Thr-Thr-Ser-Glu-Leu (SEQ ID NO: 7)
Val-Thr-Thr-Ser-Glu-Leu-Gly (SEQ ID NO: 8), or
a peptide resistant to proteolysis defined as being derived from sequence (I) by modifications of N-terminal end in the form of an acetyl, and/or of the C-terminal end in the form of an amide group and/or at least one peptide bond modified and replaced with a (CH₂NH) bond, an (NHCO) bond, a (CH₂-O) bond, a (CH₂-S) bond, a (CH₂CH₂) bond, a (CO-CH₂) bond, a (CHOH-CH₂) bond, an (N-N) bond, an alkene-E bond or a -CH=CH bond, or a peptide homologous peptide defined as being derived from sequence (I) by one or more conservative substitution(s) and presenting more than 80% of the amino acids identical to sequence (I).

2. Peptide according to claim 1, in which X₁ is a lysine residue, X₂ is a threonine residue and X₃ is an aspartate residue.

3. Peptide according to claim 2, consisting in sequence SNFGSRKFSYKAKFWTDVTTSELG (SEQ ID NO: 2).

4. Pharmaceutical composition comprising a peptide according to one of claims 1 to 3 as active ingredient, together with one or more pharmaceutically acceptable excipients.

5. Use of a peptide according to claims 1 to 3, for modulating cell proliferation *in vitro.*

6. Peptide according to one of claims 1 to 3, as a medicinal product.

7. Peptide according to one of claims 1 to 3, for use at an efficient dose for stimulating IGF-1 synthesis and/or IGF-1 receptor expression.

8. Peptide according to claim 7, used *in vivo* in an efficient daily dose of less than 100 µg, preferably from 10 µg to 100 µg, more preferably from 10 µg to 50 µg.

9. Peptide according to one of claims 1 to 3, for use as a growth hormone substitute.

10. Peptide according to one of claims 1 to 3, for use in the treatment of chronic renal insufficiency, Turner's syndrome or Prader-Willi syndrome, or in the therapeutic treatment of metabolic syndrome, of autoimmune and/or neurodegenerative diseases.

11. Peptide according to one of claims 1 to 3, for use as neurotrophic and/or neuroprotective agent.

12. Peptide according to one of claims 1 to 3, for use at a suitable dose for stimulating IGF-1 synthesis and/or IGF-1 receptor expression

13. Peptide according to claim 12, for use *in vivo* in an effective daily dose of at least 300 µg per day, preferably from 300 µg to 3 mg per day, more preferably from 1 mg to 3 mg, even more preferably from 2 mg to 3 mg.

14. Peptide according to one of claims 1 to 3, for use as a somatostatin substitute.

15. Peptide according to one of claims 1 to 3, for use in the treatment of Alzheimer's disease, or for use in the treatment of acromegaly or cancer, in particular in the treatment of metastases.
